(19) Europäisches Patentamt
European Patent Office
Office européen des brevets

(11) **EP 4 447 000 A1**

(12) **EUROPEAN PATENT APPLICATION**
published in accordance with Art. 153(4) EPC

(43) Date of publication:
**16.10.2024 Bulletin 2024/42**

(51) International Patent Classification (IPC):
***G06V 10/764*** (2022.01)     ***G06T 7/10*** (2017.01)

(21) Application number: **23799145.0**

(22) Date of filing: **29.03.2023**

(86) International application number:
**PCT/CN2023/084721**

(87) International publication number:
**WO 2023/213158 (09.11.2023 Gazette 2023/45)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **06.05.2022   CN 202210486701**

(71) Applicant: **Tencent Technology (Shenzhen)
Company Limited
Shenzhen, Guangdong 518057 (CN)**

(72) Inventors:
• **XU, Zhe
Shenzhen, Guangdong 518057 (CN)**
• **LU, Donghuan
Shenzhen, Guangdong 518057 (CN)**
• **ZHENG, Yefeng
Shenzhen, Guangdong 518057 (CN)**

(74) Representative: **Nederlandsch Octrooibureau
P.O. Box 29720
2502 LS The Hague (NL)**

(54) **MEDICAL IMAGE SEGMENTATION METHOD AND APPARATUS, DEVICE, STORAGE MEDIUM, AND PROGRAM PRODUCT**

(57)   Disclosed are a medical image segmentation method and apparatus, a device, a storage medium, and a program product, which relate to the field of artificial intelligence. The method includes: performing image segmentation on a sample medical image through a source domain segmentation model, to obtain a first segmentation result; performing image segmentation on the sample medical image through a target domain segmentation model, to obtain a second segmentation result; adjusting the first segmentation result based on the second segmentation result and a segmentation confidence level of the target domain segmentation model, to obtain an adjusted segmentation result; and train the target domain segmentation model based on the second segmentation result and the adjusted segmentation result so as to update model parameters of the target domain segmentation model. The method provided in the embodiments of this application can realize passive and unsupervised domain adaptation and improve image segmentation accuracy.

EP 4 447 000 A1

Perform image segmentation on a sample medical image through a source domain segmentation model, to obtain a first segmentation result, the source domain segmentation model being obtained through training based on medical image data in a source domain, the sample medical image being an unannotated medical image in a target domain, data distributions of medical images in the target domain and the source domain being different — 401

Perform image segmentation on the sample medical image through a target domain segmentation model, to obtain a second segmentation result — 402

Adjust the first segmentation result based on the second segmentation result and a segmentation confidence level of the target domain segmentation model, to obtain an adjusted segmentation result — 403

train the target domain segmentation model based on the second segmentation result and the adjusted segmentation result so as to update model parameters of the target domain segmentation model — 404

FIG. 4

**Description**

RELATED APPLICATION

This application claims priority to Chinese Patent Application No. 202210486701.5, entitled "MEDICAL IMAGE SEGMENTATION METHOD AND APPARATUS, DEVICE, STORAGE MEDIUM, AND PROGRAM PRODUCT" and filed on May 06, 2022, which is incorporated herein by reference in its entirety.

FIELD OF THE TECHNOLOGY

Embodiments of this application relate to the field of artificial intelligence (AI), and in particular, to a medical image segmentation method and apparatus, a device, a storage medium, and a program product.

BACKGROUND OF THE DISCLOSURE

Currently, image segmentation technologies based on AI may be applied to the medical field, for example, performing image segmentation on fundus images to obtain optic cup and optic disc segmentation results.
During image segmentation, a pre-trained image segmentation model may be used for training. When the pre-trained image segmentation model is used for image segmentation, because data used by the pre-trained image segmentation model and image data that currently needs to be segmented belong to different domains, for example, from different medical centers or from different imaging instruments, the pre-trained image segmentation model needs to be further trained, to adapt to the image data in the target domain. In the related art, during domain adaptation, source domain data used by the image segmentation model during pre-training needs to be used, to adapt the pre-trained image segmentation model to the target domain.
That is, in the related art, domain adaptation relies on the source domain data. In a case that the source domain data cannot be acquired, domain adaptation cannot be performed, which affects the effect of the image segmentation model on target domain image segmentation.

SUMMARY

Embodiments of this application provide a medical image segmentation method and apparatus, a device, a storage medium, and a program product, which can realize passive and unsupervised domain adaptation and improve image segmentation accuracy. The technical solutions are as follows:
According to an aspect, an embodiment of this application provides a medical image segmentation method, performed by a computer device, the method including:

performing image segmentation on a sample medical image through a source domain segmentation model, to obtain a first segmentation result, the source domain segmentation model being obtained through training based on medical image data in a source domain, the sample medical image being an unannotated medical image in a target domain, a data distribution of medical image data of the sample medical image in the target domain and a distribution of medical image data in the source domain being different;

performing image segmentation on the sample medical image through a target domain segmentation model, to obtain a second segmentation result, wherein initial model parameters of the target domain segmentation model are the same as model parameters of the source domain segmentation model;

adjusting the first segmentation result based on the second segmentation result and a segmentation confidence level of the target domain segmentation model, to obtain an adjusted segmentation result; and

training the target domain segmentation model based on the second segmentation result and the adjusted segmentation result so as to update model parameters of the target domain segmentation model.

According to another aspect, an embodiment of this application provides a medical image segmentation apparatus, including:

an image segmentation module, configured to perform image segmentation on a sample medical image through a source domain segmentation model, to obtain a first segmentation result, the source domain segmentation model being obtained through training based on medical image data in a source domain, the sample medical image being an unannotated medical image in a target domain, a data distribution of medical image data of the sample medical image in the target domain and a distribution of medical image data in the source domain being different;

the image segmentation module being further configured to perform image segmentation on the sample medical image through a target domain segmentation model, to obtain a second segmentation result, wherein initial model parameters of the target domain segmentation model are the same as model parameters of the source domain segmentation model;

an adjustment module, configured to adjust the first segmentation result based on the second segmentation result and a segmentation confidence level of the target domain segmentation model, to obtain an adjusted segmentation result; and

a training module, configured to train the target domain segmentation model based on the second segmentation result and the adjusted segmentation result so as to update model parameters of the target domain segmentation model.

According to another aspect, an embodiment of this application provides a computer device, including a processor and a memory, the memory storing at least one instruction, at least one program, a code set, or an instruction set, and the at least one instruction, the at least one program, the code set, or the instruction set being loaded and executed by the processor to implement the medical image segmentation method according to the foregoing aspects.

According to another aspect, a computer-readable storage medium is provided, the readable storage medium storing at least one instruction, at least one program, a code set, or an instruction set, the at least one instruction, the at least one program, the code set, or the instruction set being loaded and executed by a processor to implement the medical image segmentation method according to the foregoing aspects.

According to another aspect, an embodiment of this application provides a computer program product or a computer program, the computer program product or the computer program including computer instructions, the computer instructions being stored in a computer-readable storage medium. A processor of a computer device reads the computer instructions from the computer-readable storage medium, and executes the computer instructions, to cause the computer device to perform the medical image segmentation method provided in the foregoing aspects.

In the embodiments of this application, in a case that a sample medical image in a target domain is unannotated, a computer device segments the sample medical image through a source domain segmentation model and a target domain segmentation model respectively, to obtain a first segmentation result and a second segmentation result. In a case that there is difference between the second segmentation result and the first segmentation result, it indicates that there may be an incorrect result in the first segmentation result, so that the second segmentation result is used to adjust the first segmentation result; and the target domain segmentation model may have an inaccurate segmentation problem, and the first segmentation result further needs to be adjusted based on a segmentation confidence level of the target domain segmentation model, to improve a confidence level of the adjusted first segmentation result, that is, an adjusted segmentation result, and use the adjusted segmentation result as a pseudo label training model, thereby helping to improve the model training accuracy.

In addition, the adjusted segmentation result and the second segmentation result are used to train the target domain segmentation model, so that the trained target domain segmentation model is adapted to the image data in the target domain, which can realize unsupervised training on the target domain segmentation model without relying on source domain data, that is, realize passive and unsupervised domain adaptation, thereby improving the accuracy of the target domain segmentation model in segmenting the target domain image data.

BRIEF DESCRIPTION OF THE DRAWINGS

FIG. 1 is a schematic principle diagram of a medical image segmentation method according to an embodiment of this application.

FIG. 2 is a schematic diagram of an application scenario according to an exemplary embodiment of this application.

FIG. 3 is a schematic diagram of an implementation environment according to an exemplary embodiment of this application.

FIG. 4 is a flowchart of a medical image segmentation method according to an exemplary embodiment of this application.

FIG. 5 is a flowchart of a medical image segmentation method according to another exemplary embodiment of this application.

FIG. 6 is a flowchart of a medical image segmentation method according to another exemplary embodiment of this application.

FIG. 7 is a schematic diagram of implementation of a training process of a target domain segmentation model according to an exemplary embodiment of this application.

FIG. 8 is a schematic diagram of segmentation results of target domain segmentation models trained under different technologies.

FIG. 9 is a structural block diagram of a medical image segmentation apparatus according to an exemplary embodiment of this application.

FIG. 10 is a schematic structural diagram of a computer device according to an exemplary embodiment of this application.

DESCRIPTION OF EMBODIMENTS

AI is a theory, method, technology, and application system that uses a digital computer or a machine controlled by the digital computer to simulate, extend, and expand human intelligence, perceive an environment, acquire knowledge, and use knowledge to obtain an optimal result. In other words, AI is a comprehensive technology of computer sciences, attempts to understand essence of intelligence, and produces a new intelligent machine that can react in a manner similar to human intelligence. The AI is to study the design principles and implementation methods of various intelligent machines, to enable the machines to have the functions of perception, reasoning, and decision-making.

An AI technology is a comprehensive discipline, covering a wide range of fields including both a hardware-level technology and a software-level technology. The basic AI technology generally includes a technology such as a sensor, a dedicated AI chip, cloud computing, distributed storage, a big data processing technology, an operation/interaction system, or mechatronics. AI software technologies mainly include several major directions such as a computer vision (CV) technology, a speech processing technology, a natural language processing technology, and machine learning/deep learning.

Computer vision (CV) is a science that studies how to use a machine to "see", and furthermore, that uses a camera and a computer to replace human eyes to perform machine vision such as recognition and measurement on a target, and further perform graphic processing, so that the computer processes the target into an image more suitable for human eyes to observe, or an image transmitted to an instrument for detection. As a scientific discipline, the CV studies related theories and technologies and attempts to establish an AI system that can obtain information from images or multidimensional data. The CV technologies generally include technologies such as image processing, image recognition, image segmentation, image semantic understanding, image retrieval, video processing, video semantic understanding, video content/behavior recognition, three-dimensional object reconstruction, a 3D technology, virtual reality, augmented reality, synchronous positioning, and map construction, and further include biological feature recognition technologies such as common face recognition and fingerprint recognition. The medical image segmentation method involved in the embodiments of this application is the application of the computer vision technology in the field of image segmentation.

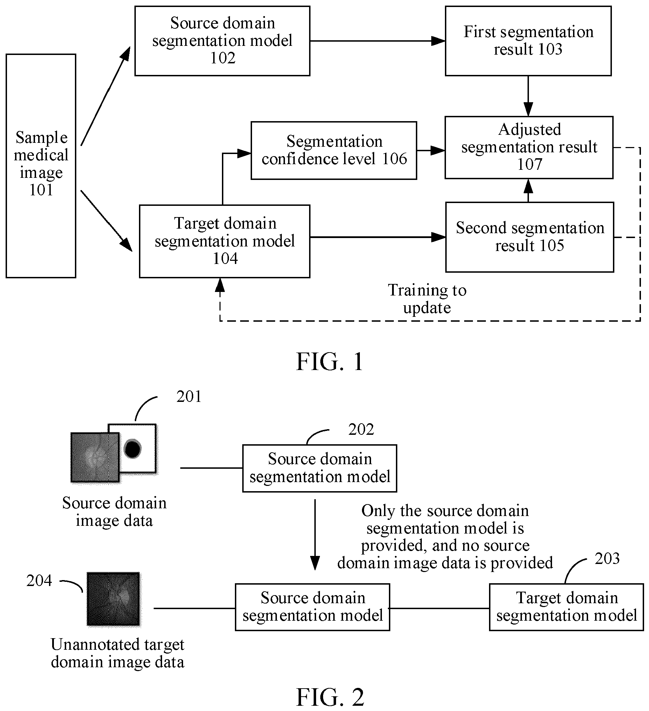

For example, as shown in FIG. 1, a computer device first performs image segmentation on a sample medical image 101 by using a source domain segmentation model 102 to obtain a first segmentation result 103, and performs image segmentation on the sample medical image 101 by using a target domain segmentation model 104 to obtain a second segmentation result 105, then adjusts the first segmentation result 103 by using the second segmentation result 105 and a segmentation confidence level 106 of the target domain segmentation model 104 to obtain an adjusted segmentation result 107. Finally, the computer device trains the target domain segmentation model 104 by using the second segmentation result 105 and the adjusted segmentation result 107 so as to update model parameters of the target domain segmentation model, to cause the target domain segmentation model to be applicable to segmentation of image data in the target domain. The first segmentation result is adjusted based on the second segmentation result through the target domain segmentation model to obtain the adjusted segmentation result, which can improve the confidence level of the adjusted segmentation result, and the target domain segmentation model is trained based on the adjusted segmentation result and the second segmentation result, to realize a supervised self-training process, causing the trained target domain segmentation model to be applicable to segmentation of target domain image data, thereby realizing domain adaptation without relying on source domain data, and improving the accuracy of target domain image segmentation.

The medical image segmentation method provided in the embodiments of this application may be applied in the training process of an image segmentation model that requires domain adaptation.

As shown in FIG. 2, when the method is applied to a medical scenario, the customer and the supplier belong to different domains, for example, different medical centers. The customer can acquire a source domain segmentation model 202 trained by using source domain image data 201 from the supplier, but cannot acquire the source domain image data (that is, the supplier only provides the source domain segmentation model and provides no source domain image data). Subsequently, the customer can train the source domain segmentation model 202 (initialized target domain segmentation model) to obtain a domain-adapted target domain segmentation model 203, to apply the target domain segmentation model 203 to segmentation of unannotated target domain image data 204.

In addition to the foregoing application scenario, the medical image segmentation method provided in the embodiments of this application may be alternatively applied to other image segmentation scenarios in which domain adaptation is required. A specific application scenario is not limited in the embodiments of this application.

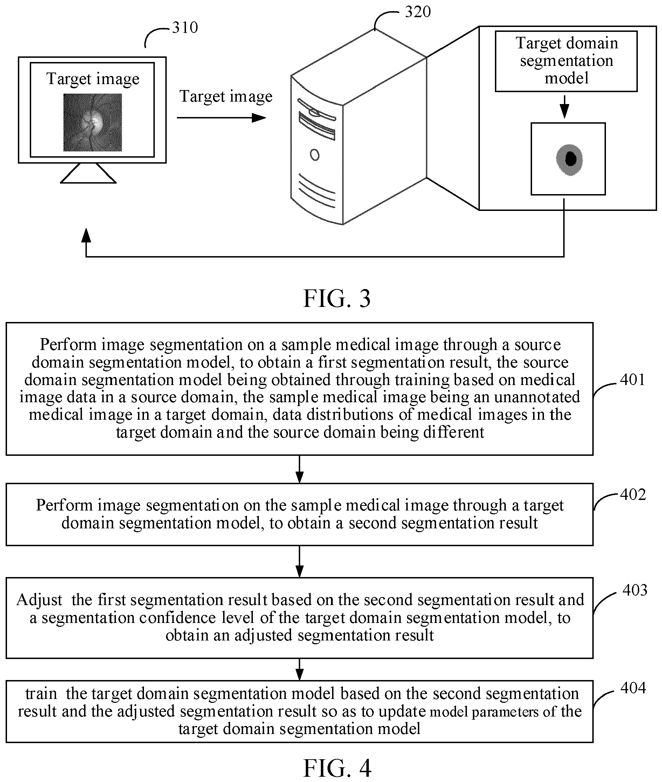

FIG. 3 is a schematic diagram of an implementation environment according to an exemplary embodiment of this application. The implementation environment includes a computer device 310 and a server 320. The computer device 310 performs data communication with the server 320 through a communication network. In some embodiments, the communication network may be a wired network or a wireless network, and the communication network may be at least one of a local area network, a metropolitan area network, or a wide area network.

The computer device 310 is an electronic device having an image segmentation requirement, and the electronic device may be a smartphone, a tablet computer, a personal computer, or the like. This is not limited in this embodiment. In FIG. 3, the computer device 310 being a computer used by medical staff is used as an example for illustration.

In some embodiments, an application supporting an image segmentation function is run on the computer device 310. When a target image needs to be segmented, the user inputs the target image into the application, and the computer device 310 may upload the target image to the server 320. The server 320 performs image segmentation and feeds back segmentation results.

The server 320 may be an independent physical server, or may be a server cluster or a distributed system formed by a plurality of physical servers, or may be a cloud server that provides basic cloud computing services such as a cloud service, a cloud database, cloud computing, a cloud function, cloud storage, a network service, cloud communication, a middleware service, a domain name service, a security service, a content delivery network (CDN), big data, and an AI platform.

In some embodiments, the server 320 is configured to provide an image segmentation service to the application run on the computer device 310. In addition, the server 320 may perform model training based on a source domain segmentation model and a sample medical images in a target domain to obtain a trained target domain segmentation model. In a possible implementation, after receiving the target image transmitted by the computer device 310, the server 320 uses the trained target domain segmentation model to perform image segmentation to obtain a target segmentation result, and returns the target segmentation result to the computer device 310, so that the computer device 310 displays the image segmentation result.

Certainly, in other possible implementations, the target domain segmentation model may alternatively be deployed on the computer device 310 side, and the computer device 310 implements image segmentation locally without the server 320. This is not limited in this embodiment. In addition, the target domain segmentation model may be trained on the server side or on the computer device side, for deployment of the target domain segmentation model. For ease of description, the following embodiments are described by using an example in which the medical image segmentation method is performed by a computer device.

FIG. 4 is a flowchart of a medical image segmentation method according to an exemplary embodiment of this application. In this embodiment, a description is made by using an example in which the method is applied to a computer device. The method includes the following operations:

Operation 401: Perform image segmentation on a sample medical image through a source domain segmentation model, to obtain a first segmentation result, the source domain segmentation model being obtained through training based on medical image data in a source domain, the sample medical image being an unannotated medical image in a target domain, data distributions of medical images in the target domain and the source domain being different.

The source domain is an image data set where image data used when training a source domain segmentation model is located. The target domain is an image data set where image data to be segmented is located. The source domain and the target domain may be, for example, image data sets from different medical centers or different imaging instruments. More specifically, for example, the source domain may be an image data set on fundus from a first medical center, and the target domain may be an image data set on fundus from a second medical center different from the first medical center. In some embodiments, the source domain segmentation model is an image segmentation model trained in advance by using image data in the source domain, and may be the initial image segmentation model in the target domain. Initial model parameters of the target domain segmentation model are the same as those of the source domain segmentation model. The source domain and the target domain are different domains, and data distributions of medical images in the source domain and the target domain are different. Because images in different domains have differences in data distributions, the initialized target domain segmentation model cannot directly segment the image data in the target domain, and domain adaptation is required, that is, the initialized target domain segmentation model (source domain segmentation model) needs to be trained, to adapt to the image data in the target domain, thereby performing image segmentation on the image data in the target domain. For example, images from different medical centers have different data distributions of medical images due to equipment parameters, brands, and the like. Therefore, when a second medical center (provider of target domain) needs to use the source domain segmentation model provided by a first medical center (provider of source domain), model adaptation is required.

In one possible case, during training of the target domain segmentation model, all the image data in the target domain has not been annotated or the amount of annotation is excessively small, which cannot be used for training; and the image data in the source domain also cannot be acquired for domain adaptation, so that in this embodiment of this application, the target domain segmentation model is trained in a passive and unsupervised domain adaptation manner.

In a possible implementation, the computer device performs image segmentation on the sample medical image by using the source domain segmentation model in advance, to obtain a first segmentation result. The first segmentation result refers to a labeling result that includes objects to which the pixels in the sample medical image belong, indicating pixel categories to which the pixels belong, which has the same size as the sample medical image. For example, when the sample medical image is a fundus image and the segmentation objects are an optic cup and an optic disc, the first segmentation result may include segmentation results for the optic cup and the optic disc respectively. The segmentation result for the optic cup is a result obtained by performing different labeling on pixels that belong to the optic cup and pixels that do not belong to the optic cup in the fundus image, thereby indicating whether the pixels belong to a pixel category corresponding to the optic cup.

In some embodiments, the model structure of segmentation model may be DeepLabv3+, U-Net, a dense convolutional network (DenseNet), a residual network (ResNet), or the like. This is not limited in this embodiment.

Operation 402: Perform image segmentation on the sample medical image through a target domain segmentation model, to obtain a second segmentation result.

In a possible implementation, the computer device uses a classification result of the sample medical image indicated in the first segmentation result as a pseudo label, and performs self-training by using the pseudo label. The source domain segmentation model may not be adapted to the sample medical image belonging to the target domain, that is, its segmentation result for the sample medical image is not accurate. Therefore, the first segmentation result needs to be adjusted first.

In the adjustment process, the computer device performs image segmentation on the sample medical image through the target domain segmentation model, to obtain a second segmentation result. The second segmentation result also includes labeling results of objects to which the pixels in the sample medical image belong, that is, the pixel categories of the pixels. The second segmentation result has the same segmentation object as the first segmentation result, for example, both being results of segmenting the optic cup in the fundus image.

Operation 403: Adjust the first segmentation result based on the second segmentation result and a segmentation confidence level of the target domain segmentation model, to obtain an adjusted d segmentation result.

After obtaining the second segmentation result, the computer device may use a difference between the second segmentation result and the first segmentation result to adjust the first segmentation result. The first segmentation result includes indication for pixel categories of the pixels in the sample medical image. In a possible implementation, the computer device may use the second segmentation result to determine that there may be an incorrect segmentation result (incorrect pixel category) in the first segmentation result, and thereby adjusting the pixel category that may be incorrect. For example, categories of a pixel A in the sample medical image indicated in the second segmentation result and the first segmentation result are not the same, indicating that the segmentation result corresponding to the pixel A in the first segmentation result may be incorrect, and the pixel category of the pixel A in the first segmentation result may be adjusted d.

In addition, in this embodiment of this application, in addition to using the second segmentation result to adjust the first segmentation result, the segmentation confidence level of the target domain segmentation model is further introduced. The segmentation confidence level of the target domain segmentation model represents an uncertainty degree of the target domain segmentation model for image segmentation, that is, the accuracy of the target domain segmentation model in segmenting the sample medical image. The second segmentation result is obtained by performing image segmentation by the target domain segmentation model, and when the target domain segmentation model performs image segmentation, there is uncertainty, that is, the second segmentation result may also not be accurate. Therefore, the segmentation confidence level is introduced and the first segmentation result is adjusted, thereby improving the confidence level of the adjusted segmentation result.

The first segmentation result is a segmentation result of the source domain segmentation model, which remains unchanged during each round of training on the target domain segmentation model. That is, during each round of training on the target domain segmentation model, the initial first segmentation result is always the result obtained by the source domain segmentation model through direct segmentation, and does not change with the training process. The model parameters of the target domain segmentation model are continuously updated in the training process, and the second segmentation result is updated accordingly. Correspondingly, the segmentation confidence level of the target domain segmentation model is also updated accordingly. During each update of the model parameters, the first segmentation result is adjusted by using the second segmentation result and the segmentation confidence level of the target domain segmentation model under the current model parameters.

Operation 404: Training the target domain segmentation model based on the second segmentation result and the adjusted segmentation result so as to update model parameters of the target domain segmentation model.

After obtaining the adjusted segmentation result, the computer device uses the adjusted segmentation result as a pseudo label for model training, to realize unsupervised training. In addition, in this process, medical image data in the source domain does not need to be used, and passive training can be realized. In a possible implementation, the computer device may use the difference between the second segmentation result and the adjusted segmentation result to train the target domain segmentation model so as to update model parameters of the target domain segmentation model until a training end condition is met.

Based on the above, in this embodiment of this application, in a case that a sample medical image in a target domain is unannotated, a computer device segments the sample medical image through a source domain segmentation model and a target domain segmentation model respectively, to obtain a first segmentation result and a second segmentation result. In a case that there is difference between the second segmentation result and the first segmentation result, it indicates that there may be an incorrect result in the first segmentation result, so that the second segmentation result is used to adjust the first segmentation result; and the target domain segmentation model may have an inaccurate segmentation problem, and the first segmentation result further needs to be adjusted based on a segmentation confidence level of the target domain segmentation model, to improve a confidence level of the adjusted first segmentation result, that is, an adjusted segmentation result, and use the adjusted segmentation result as a pseudo label training model, thereby helping to improve the model training accuracy.

In addition, the adjusted segmentation result and the second segmentation result are used to train the target domain segmentation model, so that the trained target domain segmentation model is adapted to the image data in the target domain, which can realize unsupervised training on the target domain segmentation model without relying on source domain data, that is, realize passive and unsupervised domain adaptation, thereby improving the accuracy of the target domain segmentation model in segmenting the target domain image data.

In a possible implementation, the first segmentation result includes the pixel categories of the pixels in the sample medical image. In the process of adjusting the first segmentation result, the incorrect pixel categories in the first segmentation result are first determined, and then the incorrect pixel categories are adjusted. The process of determining the incorrect pixel categories is exemplarily described below.

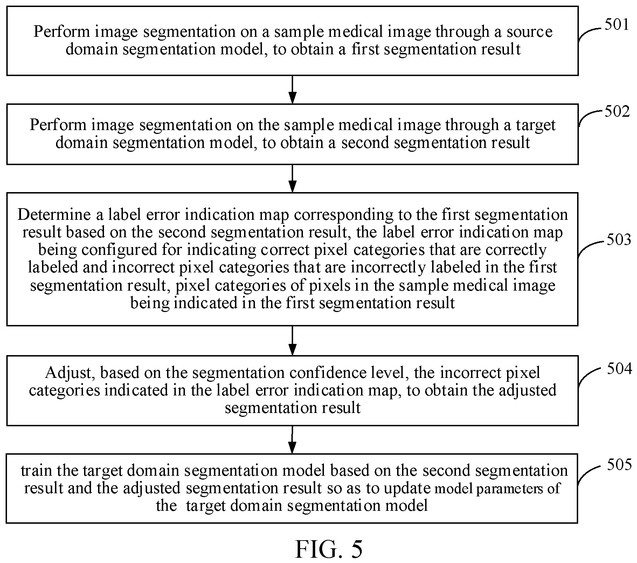

FIG. 5 is a flowchart of a medical image segmentation method according to another exemplary embodiment of this application. In this embodiment, a description is made by using an example in which the method is applied to a computer device. The method includes the following operations:

Operation 501: Perform image segmentation on a sample medical image through a source domain segmentation model, to obtain a first segmentation result.

Operation 502: Perform image segmentation on the sample medical image through a target domain segmentation model, to obtain a second segmentation result.

For the implementation of operations 501 and 502, reference may be made to operations 401 and 402, and details are not described again in this embodiment.

Operation 503: Determine a label error indication map corresponding to the first segmentation result based on the second segmentation result, the label error indication map being configured for indicating correct pixel categories that are correctly labeled and incorrect pixel categories that are incorrectly labeled in the first segmentation result, pixel categories of pixels in the sample medical image being indicated in the first segmentation result.

In a possible implementation, the computer device uses the difference between the second segmentation result and the first segmentation result to determine the incorrect pixel categories and the correct pixel categories, that is, incorrect labels and correct labels, in the pixel categories of the pixels indicated in the first segmentation result, thereby obtaining a label error indication map. Different pixel categories correspond to different label error indication maps. For example, when the sample medical image is a fundus image, the pixel categories include optic cup, optic disc, and the like. The optic cup and optic disc may respectively correspond to different label error indication maps. The label error indication map corresponding to the optic cup is configured for indicating incorrect labeling and correct labeling in the pixel categories labeled as optic cup and labeled as non-optic cup; and the label error indication map corresponding to the optic disc is configured for indicating incorrect labeling and correct labeling in the pixel categories labeled as optic disc and labeled as non-optic disc. In addition, the label error indication map has the same image size as the sample medical image, and includes indication of whether the pixel categories in the sample medical image are incorrectly labeled. In some embodiments, this operation may include operation 503a to operation 503c (not shown in the figure): Operation 503a: Determine, based on the first segmentation result, first pixels belonging to a first category.

The computer device may use the first segmentation result as a pseudo label to train the target domain segmentation model. First, the computer device determines, based on the first segmentation result, probabilities that the pixels in the sample medical image belong to the first category. Because the first segmentation result contains noise, the first segmentation result needs to be filtered. That is, the first segmentation result needs to be denoised, and the denoised result is used as a pseudo label. In a possible implementation, filtering is performed in an adaptive class-perception manner, that is, different filtering thresholds are set for different pixel categories to avoid the problem of unbalanced label filtering caused by using a category-independent threshold for filtering.

After obtaining the first segmentation result, the computer device may use the first segmentation result to determine first pixels belonging to the first category. The first segmentation result includes probabilities that the pixels belong to the first category, and the computer device may use the probabilities that the pixels belong to the first category to determine whether a pixel belongs to the first category. The manner may include the following operations:

Operation 1: Determine, based on the first segmentation result, probabilities that the pixels in the sample medical image belong to the first category.

In some embodiments, the computer device may determine, based on predicted probabilities indicated in the first segmentation result, the probabilities that the pixels belong to the first category. The predicted probabilities indicated in the first segmentation result includes the probabilities that the pixels belong to different categories. For example, during segmentation of the fundus image, the first segmentation result may include probabilities that the pixels belong to the optic cup and the optic disc. When the first category is the optic cup, the computer device may acquire, according to the first segmentation result, the probabilities that the pixels belong to the optic cup.

Operation 2: Determine a first probability threshold corresponding to the first category based on the probabilities that the pixels belong to the first category.

In a possible implementation, the first probability threshold of the first category is determined based on the probabilities that the pixels belong to the first category, to screen the pixels belonging to the first category. The process of determining the first probability threshold may include the following operations:

Operation 1: Determine a maximum probability value in the probabilities that the pixels belong to the first category.

The computer device determines the maximum probability value in the probabilities that the pixels belong to the first category. For example, when the first category is the optic cup, the computer device determines the probabilities that the pixels in the sample medical image belong to the optic cup, and determines the maximum value in the probabilities.

Operation 2: Determine the first probability threshold based on the maximum probability value.

After obtaining the maximum probability value, the computer device may lower the maximum probability value by a target proportion, to obtain the first probability threshold. In some embodiments, the computer device may determine the first probability threshold by using a preset probability parameter (that is, a target proportion for adjustment) and the determined maximum probability value. The manner is as follows:

$$\hat{\gamma}^c = \alpha \cdot max(\mathbf{P}^c)$$

where $\mathbf{P}^c$ represents a set of the probabilities that the pixels belong to the first category, and $\alpha$ is the probability parameter. In some embodiments, $\alpha$ is 0.75.

Operation 3: Determine, in a case that a probability that a pixel belongs to the first category is greater than the first probability threshold, the pixel as a first pixel.

The first probability threshold is configured for filtering probabilities of low confidence levels in the first segmentation result. When the probability that a pixel belongs to the first category is greater than the first probability threshold, it is determined that the confidence level is relatively high, and the pixel may be determined as a first pixel, that is, it is determined that the pixel belongs to the first category. That is:

$$\bar{y}_t^c = \mathbb{1}[p^c > \hat{\gamma}^c]$$

where $p^c$ is the probability that the pixel belongs to the first category,

$$\mathbb{1}[p^c > \hat{\gamma}^c]$$

is an indicator function, when $p^c > \hat{\gamma}^c$, $\bar{y}_t^c$ is 1, indicating that the pixel belongs to the first category; and when $p^c < \hat{\gamma}^c$, $\bar{y}_t^c$ is 0, indicating that the pixel does not belong to the first category.

The above is only an exemplary description of the first pixels determined to belong to the first category. Similarly, pixels belonging to other categories in the first segmentation result may also be determined in the same manner.

Operation 503b: Determine, based on the second segmentation result, potential probabilities that the first pixels belong to a second category, the second category being a pixel category different from the first category.

In the foregoing process, the first segmentation result is initially screened based on the threshold policy, which is only a rough denoising process. By performing rough denoising on the first segmentation result, pseudo labels are obtained. To improve the quality of pseudo labels, fine denoising further needs to be performed. The fine denoising process is described below.

First, the computer device uses the segmentation result of the target domain segmentation model as a cross-validation third party to finely adjust the pseudo labels. In a possible implementation, the class conditional classification noise process (CNP) hypothesis is used, that is, it is assumed that each pixel in the sample medical image has a correct (potential) category label, and each category label has a certain probability of being incorrectly labeled as another category label, that is, each category label has a certain probability of being labeled as another category label in the pseudo label. For example, the potential label of a pixel $x_{t(w,h)} \in \mathbf{X}_t$ is $y_{t(w,h)}^*$, and each label belonging to a category $j$ may be labeled as a category $i$ in the pseudo label with a probability $p(\bar{y}_t = i \mid y_t^* = j)$, where $i \in C$, and $j \in C$, and $C$ is the set of pixel categories in the sample medical image.

In a possible implementation, the target domain segmentation model may output out-of-sample predicted probabilities (the second segmentation result), which is the probabilities that the pixels in the sample medical image belong to different pixel categories obtained after the target domain segmentation model segments the sample medical image. When the second segmentation result indicates that the probability that a pixel belongs to a target category is greater than a certain threshold, it means that the pixel may belong to the target category. For example, when the pseudo label of $x_t$ meets $\bar{y}_t = i$, and the probability that the pixel $x_t$ belongs to a category $j$ indicated in the second segmentation result is greater than the probability threshold ($\hat{p}_t^j(x_t) \geq \gamma^j$), the potential label of the pixel $x_t$ may be $j$ instead of $i$.

Therefore, the computer device may determine potential labels of the pixels according to the second segmentation result, thereby determining whether there is an incorrect label in the pseudo labels. In some embodiments, for first pixels of which pseudo labels are the first category, the computer device may acquire a potential probability that a target pixel belongs to the second category in the second segmentation result, the second category being a pixel category different from the first category, thereby determining, according to the potential probability, the possibility that the potential label of the pixel is the second category and the pseudo label thereof is the first category.

For example, when the first category is the optic cup, the computer device may acquire the probability that the first pixel belongs to a non-optic cup category (for example, the optic disc) in the second segmentation result.

Operation 503c: Determine, based on the potential probabilities, the label error indication map corresponding to the first category in the first segmentation result.

After obtaining the potential probabilities, the computer device may determine a label error indication map corresponding to the first category based on the potential probabilities. This operation may include the following operations:

Operation 1: Determine a second probability threshold of the second category based on probabilities that the pixels belong to the second category in the second segmentation result.

When the second segmentation result indicates that the probability that a pixel belongs to the second category is greater than the corresponding second probability threshold, it can be determined that a potential label of the pixel may be the second category. The computer device first determines the second probability threshold corresponding to the second category. In a possible implementation, an average value of the probabilities that the pixels belong to the second category may be determined as the second probability threshold. That is:

$$\gamma^j = \text{mean}\left(\hat{\mathbf{P}}_t^j\right)$$

where $\widehat{\mathbf{P}}_t^j$ is a set of the probabilities that the pixels belong to the second category in the second segmentation result.

Alternatively, in another possible implementation, the second probability threshold may be determined according to the maximum probability value in the probabilities that the pixels belong to the second category. That is:

$$\gamma^j = \alpha \cdot \max\left(\widehat{\mathbf{P}}_t^j\right)$$

where $\alpha$ is a probability parameter.

Operation 2: Determine a joint distribution matrix based on the potential probabilities and the second probability threshold, the joint distribution matrix being configured for indicating probabilities that pixels are labeled as the first category and a potential category of the pixels is the second category.

In a possible implementation, the computer device uses the second probability threshold for filtering, to obtain the quantity of possible pixels of which potential labels are the second category and pseudo labels are the first category (that is, the quantity of first pixels indicated to be of the second category in the second segmentation result), thereby further determining, according to the quantity, the probabilities that the pseudo labels of the pixels in the sample medical image are the first category and the potential category thereof is the second category, that is, the joint distribution matrix. The process of determining the joint distribution matrix may include the following operations:

Operation 1: Determine a confidence joint matrix based on the potential probabilities and the second probability threshold, the confidence joint matrix being configured for indicating a quantity of the first pixels of which the potential category is the second category.

First, the computer device may construct a confidence joint matrix by using the potential probabilities and the second probability threshold, where the manner is as follows:

$$\mathbf{CJ}_{\bar{y}_t, y_t^*}[i][j] = \left|\mathbf{X}_{t\left(\bar{y}_t^i, y_t^*\right)}\right| = \left|\left\{x_t \in \mathbf{X}_{t(\bar{y}_t^i)} : \hat{p}_t^j(x_t) \geq \gamma^j, j = \underset{c \in C: \hat{p}_t^c(x_t) \geq \gamma^c}{\arg\max} \hat{p}_t^c(x_t)\right\}\right|$$

where $\mathbf{CJ}_{\bar{y}_t, y_t^*}[i][j]$ represents the quantity of first pixels of which the pseudo labels are a first category i, and the potential labels thereof are a second category j, $\mathbf{X}_{t(\bar{y}_t^i)}$ represents first pixels of which the pseudo labels are the first category i in the sample medical image, and $\hat{p}_t^j(x_t)$ represents a probability that a pixel $x_t$ belongs to the second category j indicated in the second segmentation result.

Operation 2: Perform normalization processing on the confidence joint matrix, to obtain the joint distribution matrix.

After the confidence joint matrix is obtained, normalization processing may be performed on the confidence joint matrix, to obtain the joint distribution matrix. However, because in the process of constructing the confidence joint matrix, the computer device filters pixels meeting $\hat{p}_t^j(x_t) < \gamma^j$, the total quantity of pixels changes, and the total quantity of pixels represented by the matrix needs to be restored, calibration processing is first performed on the confidence joint matrix, where the manner is as follows:

$$\mathbf{CJ}_{\bar{y}_t, y_t^*}^*[i][j] = \frac{\mathbf{CJ}_{\bar{y}_t, y_t^*}[i][j]}{\sum_{j \in C} \mathbf{CJ}_{\bar{y}_t, y_t^*}[i][j]} \cdot \left|\mathbf{X}_{t(\bar{y}_t = i)}\right|$$

where $|\mathbf{X}_{t(\bar{y}_t = i)}|$ is the total quantity of first pixels with the pseudo label i.

Subsequently, normalization processing is performed on $\mathbf{CJ}_{\bar{y}_t, y_t^*}^*[i][j]$ obtained after the calibration processing, to obtain the joint distribution matrix, where the manner is as follows:

$$JD_{\bar{y}_t, y_t^*}[i][j] = \frac{CJ_{\bar{y}_t, y_t^*}^*[i][j]}{\sum_{i \in C, j \in C} CJ_{\bar{y}_t, y_t^*}^*[i][j]}$$

Operation 3: Determine the label error indication map corresponding to the first category based on the joint distribution matrix.

In some embodiments, the joint distribution matrix represents the probabilities that the pseudo labels indicated in the first segmentation result are the first category and the potential category of the pixels is the second category. The computer device may use the joint distribution matrix to determine the label error indication map corresponding to the first category in the first segmentation result, that is the incorrectly labeled pixels for the first category in the pseudo labels. The manner may include the following operations:
Operation 1: Determine a pixel label error quantity based on the joint distribution matrix and a total quantity of the pixels in the sample medical image.

The non-diagonal value in the joint distribution matrix represents the possibility that the first category is incorrectly labeled. Therefore, in a possible implementation, the pixel label error quantity that may exist in the first segmentation result may be determined based on the total quantity of pixels and the joint distribution matrix, that is, the pixel label error quantity may be:

$$n \cdot \sum_{j \in C, j \neq i} \left( JD_{\bar{y}_t, y_t^*}[i][j] \right)$$

where n is the total quantity of pixels in the sample medical image.

Operation 2: Select pixels with incorrect labels from the pixels in the sample medical image based on the pixel label error quantity, probabilities that the pixels with incorrect labels belong to the first category being lower than probabilities that other pixels belong to the first category.

Subsequently, the computer device selects pixels with incorrect labels from the pixels in the sample medical image based on the pixel label error quantity. In a possible implementation, the computer device sorts the probabilities that the pixels belong to the first category in the first segmentation result from low to high, and determines the top k pixels as pixels with incorrect labels, where k is the pixel label error quantity.

Operation 3: Perform first labeling on the pixels with incorrect labels and performing second labeling on the other pixels, to obtain the label error indication map.

The label error indication map of the first category includes error label indication of the pixels in the sample medical image being labeled as the first category or non-first category in the first segmentation result. In a possible implementation, the computer device may perform first labeling on selected pixels with incorrect labels, to indicate that pseudo labels of the pixels are incorrect, and perform second labeling on pixels other than the pixels with incorrect labels, indicating that pseudo labels of the pixels are correct. In some embodiments, the first labeling may be "1", and the second labeling may be "0".

In the foregoing process, the process of determining the label error indication map of the first category is described. Correspondingly, label error indication maps of other categories may be determined based on the same manner, so that the first segmentation result is adjusted respectively based on the label error indication maps corresponding to all the categories.

Operation 504: Adjust, based on the segmentation confidence level, the incorrect pixel categories indicated in the label error indication map, to obtain the adjusted segmentation result.

After acquiring the label error indication map, the computer device may determine whether to perform adjusted according to the indicated error status of the pseudo labels of the pixels. In a case that the label error indication map indicates that the pixel category is an incorrect pixel category, the pseudo label corresponding to the pixel is adjusted, and in a case that the label error indication map indicates that the pixel category is a correct pixel category, the pseudo label is determined as a label of the pixel.

For example, for the label error indication map of the first category, the first segmentation result indicates that a pixel A and a pixel B are of the first category, that is, pseudo labels of the pixel A and the pixel B are the first category, and the label error indication map indicates that the pixel category of the pixel A is an incorrect pixel category, and the pixel category of the pixel B is a correct pixel category, then it means that the pixel category of the pixel A may not be the first category and needs to be adjusted, while the pixel category of the pixel B is the first category and does not need to be adjusted.

During adjustment, because the target domain segmentation model may still have a domain offset, that is, be not adapted to the target domain data, the second segmentation result also contains noise and needs to be further adjusted based on the segmentation confidence level of the target domain segmentation model. For the process of adjusting the incorrect pixel categories based on the segmentation confidence level, reference may be made to the following embodiments.

Operation 505: Training the target domain segmentation model based on the second segmentation result and the adjusted segmentation result so as to update model parameters of the target domain segmentation model.

For the implementation of this operation, reference may be made to operation 404, and details are not described again in this embodiment.

In this embodiment of this application, first, rough denoising is performed on the first segmentation result to obtain pseudo labels for training, which can improve the accuracy of the pseudo labels. In addition, incorrect pseudo labels are determined further based on the differences between the pixel categories corresponding to the pixels in the second segmentation result and the pixel categories indicated by the pseudo labels, and the segmentation confidence level of the target domain segmentation model is used to further adjust the incorrect pseudo labels, that is, double adjustment is performed on the pseudo labels after rough denoising based on the second segmentation result and the segmentation confidence level of the target domain segmentation model, to realize fine adjustment on the pseudo labels, to further improve the accuracy of the pseudo labels, thereby improving the accuracy of model training.

In this embodiment of this application, in the process of adjusting the first segmentation result, the segmentation confidence level of the target domain segmentation model is also introduced. The process of determining the segmentation confidence level of the target domain segmentation model and the specific adjustment process will be exemplarily described below.

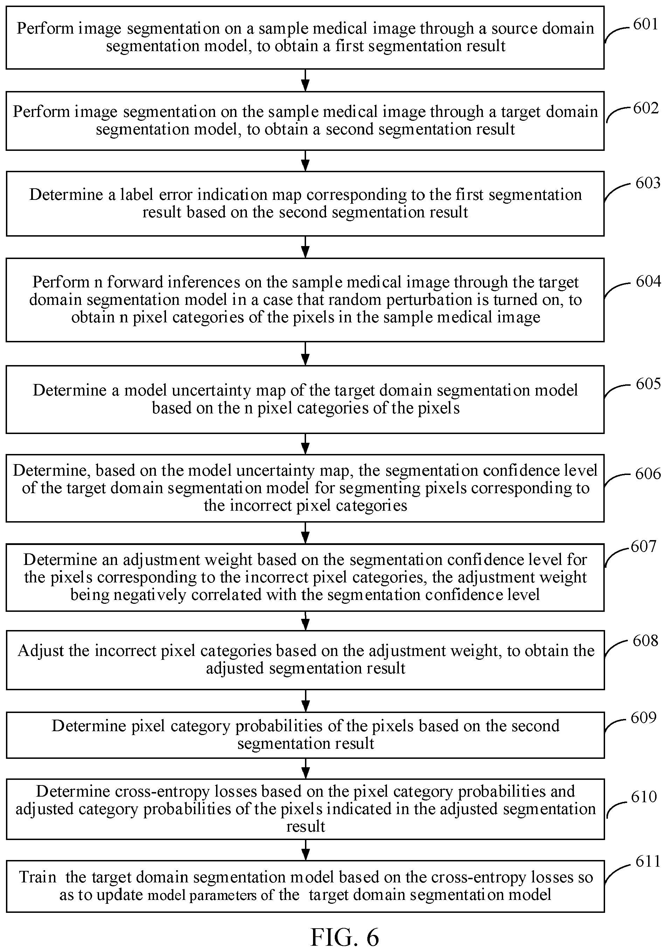

FIG. 6 is a flowchart of a medical image segmentation method according to another exemplary embodiment of this application. In this embodiment, a description is made by using an example in which the method is applied to a computer device. The method includes the following operations:

Operation 601: Perform image segmentation on a sample medical image through a source domain segmentation model, to obtain a first segmentation result.

Operation 602: Perform image segmentation on the sample medical image through a target domain segmentation model, to obtain a second segmentation result.

Operation 603: Determine a label error indication map corresponding to the first segmentation result based on the second segmentation result.

For the implementation of operation 601 to operation 603, reference may be made to the foregoing embodiments, and details are not described again in this embodiment.

Operation 604: Perform n forward inferences on the sample medical image through the target domain segmentation model in a case that random perturbation is turned on, to obtain n pixel categories of the pixels in the sample medical image.

In a possible implementation, the computer device uses segmentation uncertainty of the target domain segmentation model to determine the segmentation confidence level. The segmentation uncertainty may be determined according to the segmentation result of the target domain segmentation model in a case of being randomly perturbed.

In some embodiments, Bayesian estimation may be performed in a Monte-Carlo (MC) dropout manner, to obtain the segmentation uncertainty. The computer device turns on random perturbation, that is, turns on random dropout, for the target domain segmentation model. In the process of turning on random dropout, the model result of the target domain segmentation model will change randomly. In this case, the target domain segmentation model is used to perform n forward inferences, that is, perform n times of image segmentation, on the sample medical image, to obtain n segmentation results, where each segmentation result includes pixel category probabilities of the pixels.

For example, 10 random forward inferences may be performed, and a dropout rate is 0.5.

Operation 605: Determine a model uncertainty map of the target domain segmentation model based on the n pixel categories of the pixels.

The computer device may respectively use n pixel categories of the pixels to determine segmentation uncertainty degrees corresponding to the pixels, thereby determining the model uncertainty map of the target domain segmentation model according to the segmentation uncertainty degrees of the pixels. In a possible implementation, the probability that a pixel belongs to each category is indicated in each pixel category. The standard deviation of the n pixel categories may be determined as a segmentation uncertainty degree corresponding to the pixel.

In some embodiments, different pixel categories correspond to different model uncertainty maps. For example, when the pixel category is the first category, the computer device may acquire n pixel category probabilities that the pixels belong to the first category, thereby obtaining the corresponding segmentation uncertainty degrees of the target domain segmentation model when segmenting the pixels of the first category, the segmentation uncertainty degrees of the pixels form the model uncertainty map of the target domain segmentation model for segmentation of the first category.

Operation 606: Determine, based on the model uncertainty map, the segmentation confidence level of the target domain segmentation model for segmenting pixels corresponding to the incorrect pixel categories.

After obtaining the model uncertainty map, the computer device may acquire the segmentation uncertainty degrees of the pixels corresponding to the incorrect pixel categories according to the model uncertainty map, and obtain the segmentation confidence level for segmenting the pixels corresponding to the incorrect pixel categories. The pixels corresponding to the incorrect pixel categories are pixels labeled as the first labeling in the label error indication map. In addition, in the process of determining the segmentation confidence level, the model uncertainty map and the label error indication map are maps corresponding to the same category. For example, the segmentation confidence level for the pixels corresponding to the incorrect pixel categories in the label error indication map corresponding to the first category to be segmented by the target domain segmentation model is determined according to the model uncertainty map corresponding to the first category. In addition, the size of the model uncertainty map is the same as the label size of the label error indication map, which both have the same size as the sample medical image. For example, when a pixel A is labeled as the first labeling in the label error indication map, it can be determined that its corresponding pixel category is an incorrect pixel category. In this case, a segmentation uncertainty degree corresponding to the pixel A can be found in the model uncertainty map, thereby obtaining a segmentation confidence level of the pixel A.

Operation 607: Determine an adjustment weight based on the segmentation confidence level for the pixels corresponding to the incorrect pixel categories, the adjustment weight being negatively correlated with the segmentation confidence level.

In a possible implementation, the incorrect pixel categories, that is, incorrect pseudo labels, are adjusted based on an adjustment weight. The adjustment weight is negatively correlated with the segmentation confidence level. A lower segmentation confidence level indicates a greater adjustment weight. In some embodiments, the adjustment weight ranges from 0 to 1.

Operation 608: Adjust the incorrect pixel categories based on the adjustment weight, to obtain the adjusted segmentation result.

The manner of adjusting the incorrect pixel categories by using the adjustment weight is:

$$\dot{\mathbf{Y}}_t^c = \overline{\mathbf{Y}}_t^c + \mathbf{E}^c \cdot (-1)^{\overline{\mathbf{Y}}_t^c} \cdot (\mathbf{1} - \mathbf{U}^c)$$

where $\overline{\mathbf{Y}}_t^c$ represents the first segmentation result, $\mathbf{E}^c$ is the label error indication map, and $\mathbf{U}^c$ is the model uncertainty map. $\mathbf{E}^c$ is 0 or 1, and when $\mathbf{E}^c$ indicates that the pseudo label error corresponding to the pixel is an incorrect pixel category, the adjustment weight $(1 - \mathbf{U}^c)$ is used for adjustment. During the adjustment, incorrect pixel categories are adjusted respectively.

For example, the first segmentation result indicates that the pixel is of the first category or non- first category, when it indicates that the pixel A is of the first category, and it is indicated in $\mathbf{E}^c$ that the pixel A has the incorrect pixel category, and a segmentation uncertainty degree corresponding to the pixel A acquired from $\mathbf{U}^c$ is 0.8, the adjusted segmentation result of the pixel A is 1-(1-0.8)=0.8.

When $\mathbf{E}^c$ indicates that the pseudo label error corresponding to the pixel is the correct pixel category, there is no need to perform adjustment, that is, the first segmentation result is the same as the adjusted segmentation result.

Operation 609: Determine pixel category probabilities of the pixels based on the second segmentation result.

In a possible implementation, the computer device uses the difference between the adjusted segmentation result and the second segmentation result to train the target domain segmentation model so as to update model parameters of the target domain segmentation model, that is, uses the adjusted segmentation result obtained by adjustment as pseudo labels to train the model. In some embodiments, cross-entropy losses may be used to update model parameters of the target domain segmentation model. First, the computer device acquires, based on the second segmentation result, pixel category probabilities that the pixels belong to different pixel categories.

Operation 610: Determine cross-entropy losses based on the pixel category probabilities and adjusted category probabilities of the pixels indicated in the adjusted segmentation result.

The computer device determines the cross-entropy losses by using the pixel category probabilities and the adjusted segmentation result. There are different corresponding adjusted segmentation results for segmentation of pixels of different categories. During determining of the cross-entropy losses, corresponding cross-entropy losses may be respectively obtained for different categories. In some embodiments, during determining of a cross-entropy loss of a target category, the cross-entropy loss corresponding to the target category may be determined based on a pixel category probability of a pixel belonging to the target category and an adjusted segmentation result corresponding to the target category. The manner of determining the cross-entropy loss is as follows:

$$\mathcal{L}_{seg} = -\sum_{v} \left[ \dot{y}_{t(v)} \cdot \log\left(p_{t(v)}\right) + \left(1 - \dot{y}_{t(v)}\right) \cdot \log\left(1 - p_{t(v)}\right) \right]$$

where $\dot{y}_{t(v)}$ is the adjusted segmentation result corresponding to a pixel v. When the label error indication map indicates that the pixel category of the pixel v is a correct pixel category, the adjusted segmentation result is a pixel category probability of the pixel v in the first segmentation result; and when the label error indication map indicates that the pixel category of the pixel v is an incorrect pixel category, the adjusted segmentation result is an adjusted category probability adjusted based on the segmentation confidence level. $p_{t(v)}$ is the pixel category probability of the pixel v in the second segmentation result.

Operation 611: Train the target domain segmentation model based on the cross-entropy losses so as to update model parameters of the target domain segmentation model.

The computer device uses algorithms such as backpropagation or gradient updating to update the model parameters of the target domain segmentation model based on the cross-entropy loss until the cross-entropy loss meets a convergence condition.

In addition to using the cross-entropy loss for update training, other losses such as L1, L2, and other loss functions may alternatively be used. This is not limited in this embodiment of this application.

In this embodiment, when random perturbation is turned on, the target domain segmentation model is used to perform n forward inferences, and the inference results are used to determine the segmentation confidence level of the target domain segmentation model. In a case that the target domain segmentation model is perturbed, more stable results of the n forward inferences indicate a higher segmentation confidence level, thereby indicating a higher confidence level of the second segmentation result. In a case that the second segmentation result indicates a pseudo label error, the pseudo label error may be adjusted, thereby improving the accuracy of the adjusted segmentation result.

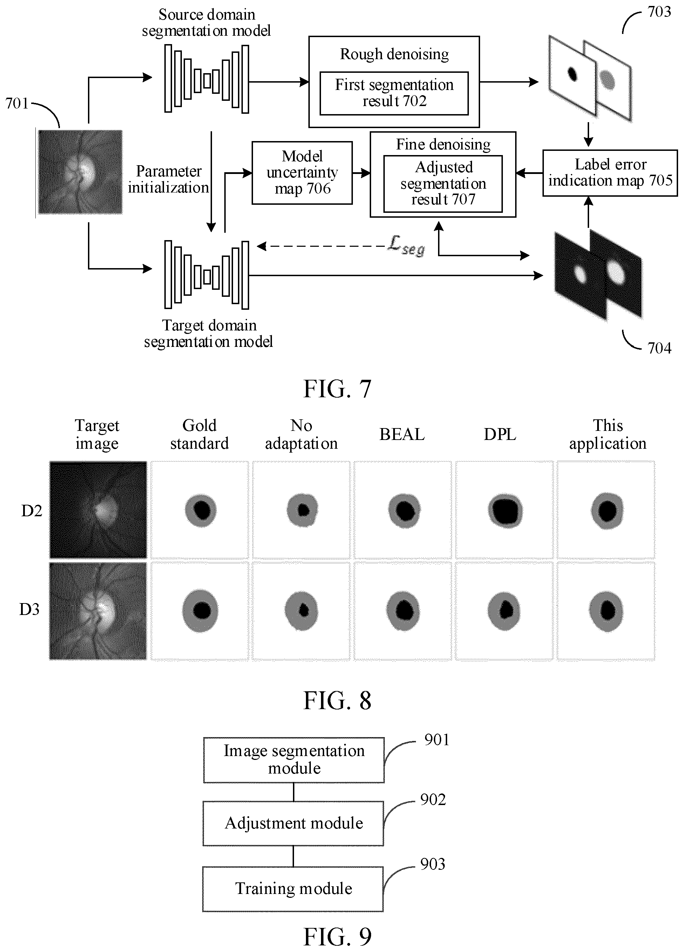

In a possible implementation, the overall structure for training the target domain segmentation model is shown in FIG. 7. The computer device inputs a sample medical image 701 into the source domain segmentation model in advance to obtain a first segmentation result 702, and subsequently performs rough denoising on the first segmentation result 702, to obtain a pseudo label 703. In addition, parameter initialization is performed on the target domain segmentation model based on the model parameters of the source domain segmentation model, to obtain an initialized target domain segmentation model. Subsequently, the computer device inputs the sample medical image 701 to the target domain segmentation model, to obtain a second segmentation result 704, so that the pseudo label 703 and the second segmentation result 704 can be used to perform label error estimation, to obtain a label error indication map 705. In addition, model uncertainty estimation further needs to be performed based on the target domain segmentation model, to obtain a model uncertainty map 706. The computer device may use the model uncertainty map 706 and the label error indication map 705 to adjust the pseudo label 703 to realize fine denoising and obtain an adjusted segmentation result 707. Finally, the adjusted segmentation result 707 and the second segmentation result 704 are used to determine a cross-entropy loss $\mathcal{L}_{seg}$, to train the target domain segmentation model by using the cross-entropy loss $\mathcal{L}_{seg}$ so as to update model parameters of the second image segmentation mod.

After the training on the target domain segmentation model is completed, the target domain image data may be segmented. In a possible implementation, image segmentation is performed on a target image belonging to the target domain through the target domain segmentation model, to obtain a target segmentation result of the target image.

Using the target domain segmentation model trained by the solution provided in the foregoing embodiments for image segmentation can improve the accuracy of segmentation results. The segmentation target being a fundus image is used as an example. The source domain segmentation model is a model pre-trained by using a Drishti-GS data set (D1), and a RIM-ONE-r3 data set (D2) and a REFUGE data set (D3) are used as target domain data.

Table 1 shows segmentation effects of target domain segmentation models corresponding to the D2 data set obtained in the domain adaptation manner in the related art and in the domain adaptation manner adopted in this embodiment of this application. Table 2 shows segmentation effects of target domain segmentation models corresponding to the D3 data set obtained in the domain adaptation manner in the related art and in the domain adaptation manner adopted in this embodiment of this application.

Table 1

| Manner | Whether to use source data (D1) | Dice (%) | | ASSD (mm) | |
|---|---|---|---|---|---|
| | | Optic cup | Optic disc | Optic cup | Optic disc |
| Upper bound | - | 78.34 (20.88) | 95.88 (2.21) | 7.51 (5.89) | 3.54 (1.82) |
| No adaptation | - | 57.33 (35.36) | 89.15 (14.07) | 16.73 (12.51) | 9.62 (9.95) |
| BEAL | Yes | 70.47 (27.40) | 90.69 (6.93) | 12.68 (14.24) | 8.90 (7.69) |
| AdvEnt | Yes | 71.23 (26.61) | 91.88 (9.87) | 10.40 (10.79) | 6.97 (5.27) |
| SRDA | Partially passive | 62.08 (21.48) | 90.62 (15.48) | 15.83 (8.51) | 8.85 (7.93) |
| TENT | No | 62.89 (19.83) | 90.25 (10.41) | 14.83 (8.62) | 8.28 (6.95) |
| DPL | No | 64.01 (19.88) | 91.48 (7.09) | 16.39 (7.53) | 8.95 (7.13) |
| This application | No | 68.59 (13.87) | 93.31 (5.41) | 11.62 (5.66) | 6.63 (6.05) |

Table 2

| Manner | Whether to use source data (D1) | Dice (%) | | ASSD (mm) | |
|---|---|---|---|---|---|
| | | Optic cup | Optic disc | Optic cup | Optic disc |
| Upper bound | - | 87.67 (6.60) | 94.79 (2.01) | 5.78 (3.67) | 4.70 (1.55) |
| No adaptation | - | 79.45 (10.74) | 89.68 (5.62) | 8.95 (4.68) | 9.03 (4.04) |
| BEAL | Yes | 83.24 (10.85) | 91.43 (3.79) | 8.24 (6.86) | 7.93 (3.85) |
| AdvEnt | Yes | 83.66 (11.03) | 90.39 (3.92) | 8.07 (7.08) | 8.68 (3.99) |
| SRDA | Partially passive | 81.57 (7.63) | 86.41 (2.93) | 8.94 (4.82) | 9.56 (4.13) |
| TENT | No | 83.58 (8.42) | 92.04 (2.37) | 8.01 (4.26) | 8.25 (4.58) |
| DPL | No | 84.02 (7.84) | 92.57 (2.81) | 7.56 (4.05) | 7.83 (3.92) |
| This application | No | 85.11 (7.77) | 93.60 (2.41) | 6.83 (4.64) | 7.09 (4.59) |

The upper bound refers to a segmentation result of the target domain segmentation model obtained by performing fully-supervised training based on labels of the target domain data. No adaptation refers to a result of directly using the source domain segmentation model to segment the target domain data. BEAL and ADVENT are segmentation results of the target domain segmentation model trained based on the source data; SRDA is a segmentation result of the target domain segmentation model partially trained based on the source data in the training process; and both TENT and DPL are segmentation results of the target domain segmentation model trained without relying on the source data. The accuracy of the segmentation results is determined based on the indicator Dice score and the average symmetric surface distance (ASSD). The data in parentheses in the table is standard deviations. The Dice score is positively correlated with the accuracy of the segmentation results, and the ASSD is negatively correlated with the accuracy of the segmentation results. It can be seen that the passive and unsupervised training manner provided in this application can improve the effect of domain adaptation of the target domain segmentation model in a case that the source data cannot be acquired and the target domain data is unannotated, thereby improving the segmentation accuracy.

FIG. 8 shows segmentation results of target images in the D2 data set and the D3 data set by target domain segmentation models obtained based on the manner in the related art and the manner of this application (where the gold standard refers to the standard result of segmentation). It can be seen that the segmentation result of this application is more accurate.

FIG. 9 is a structural block diagram of a medical image segmentation apparatus according to an exemplary embodiment of this application. As shown in FIG. 9, the apparatus includes:

an image segmentation module 901, configured to perform image segmentation on a sample medical image through a source domain segmentation model, to obtain a first segmentation result, the source domain segmentation model being obtained through training based on image data in a source domain, the sample medical image being an unannotated image in a target domain, the target domain and the source domain being different;

the image segmentation module 901 being further configured to perform image segmentation on the sample medical image through a target domain segmentation model, to obtain a second segmentation result;

an adjustment module 902, configured to adjust the first segmentation result based on the second segmentation result and a segmentation confidence level of the target domain segmentation model, to obtain an adjusted segmentation result; and

a training module 903, configured to train the target domain segmentation model based on the second segmentation result and the adjusted segmentation result so as to update model parameters of the target domain segmentation model.

In some embodiments, the adjustment module 902 is further configured to:

determine a label error indication map corresponding to the first segmentation result based on the second segmentation result, the label error indication map being configured for indicating correct pixel categories that are correctly labeled and incorrect pixel categories that are incorrectly labeled in the first segmentation result, pixel categories of pixels in the sample medical image being indicated in the first segmentation result; and

adjust, based on the segmentation confidence level, the incorrect pixel categories indicated in the label error indication map, to obtain the adjusted segmentation result.

In some embodiments, the adjustment module 902 is further configured to:

determine, based on the first segmentation result, first pixels belonging to a first category;

determine, based on the second segmentation result, potential probabilities that the first pixels belong to a second category, the second category being a pixel category different from the first category; and

determine, based on the potential probabilities, the label error indication map corresponding to the first category in the first segmentation result.

In some embodiments, the adjustment module 902 is further configured to:

determine, based on the first segmentation result, probabilities that the pixels in the sample medical image belong to the first category;

determine a first probability threshold corresponding to the first category based on the probabilities that the pixels belong to the first category; and

determine, in a case that a probability that a pixel belongs to the first category is greater than the first probability threshold, the pixel as a first pixel.

In some embodiments, the adjustment module 902 is further configured to:

determine a maximum probability value in the probabilities that the pixels belong to the first category; and

determine the first probability threshold based on the maximum probability value.

In some embodiments, the adjustment module 902 is further configured to:

determine a second probability threshold of the second category based on probabilities that the pixels belong to the second category in the second segmentation result;

determine a joint distribution matrix based on the potential probabilities and the second probability threshold, the joint distribution matrix being configured for indicating probabilities that pixels are labeled as the first category and a potential category of the pixels is the second category; and

determine the label error indication map corresponding to the first category based on the joint distribution matrix.

In some embodiments, the adjustment module 902 is further configured to:

determine a confidence joint matrix based on the potential probabilities and the second probability threshold, the confidence joint matrix being configured for indicating a quantity of the first pixels of which the potential category is the second category; and

perform normalization processing on the confidence joint matrix, to obtain the joint distribution matrix.

In some embodiments, the adjustment module 902 is further configured to:

determine a pixel label error quantity based on the joint distribution matrix and a total quantity of the pixels in the sample medical image;

select pixels with incorrect labels from the pixels in the sample medical image based on the pixel label error quantity, probabilities that the pixels with incorrect labels belong to the first category being lower than probabilities that other pixels belong to the first category; and

perform first labeling on the pixels with incorrect labels and perform second labeling on the other pixels, to obtain the label error indication map.

In some embodiments, the apparatus further includes:

a forward inference module, configured to perform n forward inferences on the sample medical image through the target domain segmentation model in a case that random perturbation is turned on, to obtain n pixel categories of the pixels in the sample medical image;

an uncertainty map determining module, configured to determine a model uncertainty map of the target domain segmentation model based on the n pixel categories of the pixels; and

a confidence level determining module, configured to determine, based on the model uncertainty map, the segmentation confidence level of the target domain segmentation model for segmenting pixels corresponding to the incorrect pixel categories.

In some embodiments, the adjustment module 902 is further configured to:

determine an adjustment weight based on the segmentation confidence level for the pixels corresponding to the incorrect pixel categories, the adjustment weight being negatively correlated with the segmentation confidence level; and

adjust the incorrect pixel categories based on the adjustment weight, to obtain the adjusted segmentation result.

In some embodiments, the training module 903 is further configured to:

determine pixel category probabilities of the pixels based on the second segmentation result;

determine cross-entropy losses based on the pixel category probabilities and adjusted category probabilities of the pixels indicated in the adjusted segmentation result; and

train the target domain segmentation model based on the cross-entropy losses so as to update model parameters of the target domain segmentation model.

Based on the above, in this embodiment of this application, in a case that a sample medical image in a target domain is unannotated, a computer device segments the sample medical image through a source domain segmentation model and a target domain segmentation model respectively, to obtain a first segmentation result and a second segmentation result. In a case that there is difference between the second segmentation result and the first segmentation result, it indicates that there may be an incorrect result in the first segmentation result, so that the second segmentation result is used to adjust the first segmentation result; and the target domain segmentation model may have an inaccurate segmentation problem, and the first segmentation result further needs to be adjusted based on a segmentation confidence level of the target domain segmentation model, to improve a confidence level of the adjusted first segmentation result, that is, an adjusted segmentation result, and use the adjusted segmentation result as a pseudo label training model, thereby helping to improve the model training accuracy.

In addition, the adjusted segmentation result and the second segmentation result are used to train the target domain segmentation model, so that the trained target domain segmentation model is adapted to the image data in the target domain, which can realize unsupervised training on the target domain segmentation model without relying on source domain data, that is, realize passive and unsupervised domain adaptation, thereby improving the accuracy of the target domain segmentation model in segmenting the target domain image data.

The apparatus provided in the foregoing embodiments is illustrated with an example of division of the foregoing functional modules. In actual application, the functions may be allocated to and completed by different functional modules according to requirements, that is, the internal structure of the apparatus is divided into different functional modules, to implement all or some of the functions described above. In addition, the apparatuses provided in the foregoing embodiments and the method embodiments fall within the same conception. For details of a specific implementation process, refer to the method embodiments. Details are not described herein again.

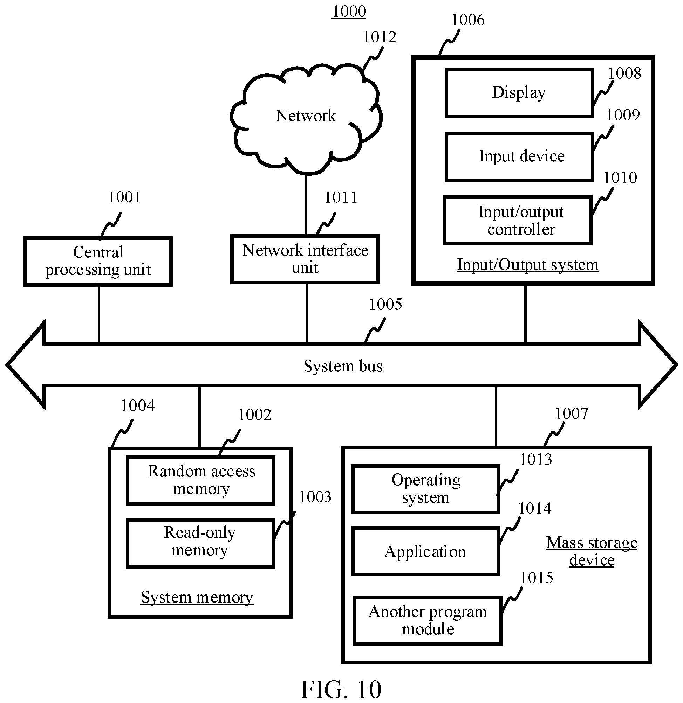

FIG. 10 is a schematic structural diagram of a computer device according to an exemplary embodiment of this application. Specifically, the computer device 1000 includes a central processing unit (CPU) 1001, a system memory 1004 including a random access memory (RAM) 1002 and a read-only memory (ROM) 1003, and a system bus 1005 connecting the system memory 1004 and the CPU 1001. The computer device 1000 further includes a basic input/output system (I/O system) 1006 configured to transmit information between components in the computer, and a mass storage device 1007 configured to store an operating system 1013, an application 1014, and another program module 1015.

The basic I/O system 1006 includes a display 1008 configured to display information and an input device 1009 such as a mouse or a keyboard that is configured for information inputting by a user. The display 1008 and the input device 1009 are both connected to the CPU 1001 by an input/output controller 1010 connected to the system bus 1005. The basic I/O system 1006 may further include the input/output controller 1010, to receive and process inputs from a plurality of other devices, such as the keyboard, the mouse, or an electronic stylus. Similarly, the input/output controller 1010 further provides an output to a display screen, a printer, or another type of output device.

The mass storage device 1007 is connected to the CPU 1001 by a mass storage controller (not shown) connected to the system bus 1005. The mass storage device 1007 and an associated computer-readable medium provide non-volatile storage for the computer device 1000. That is, the mass storage device 1007 may include a computer-readable medium (not shown), such as a hard disk or a drive.

In general, the computer-readable medium may include a computer storage medium and a communication medium. The computer-storage medium includes volatile and non-volatile media, and removable and non-removable media implemented by using any method or technology used for storing information such as computer-readable instructions, data structures, program modules, or other data. The computer storage medium includes a RAM, a ROM, a flash memory or another solid-state storage technology, a compact disc read-only memory (CD-ROM), a digital versatile disc (DVD) or another optical storage, a magnetic cassette, a magnetic tape, or a magnetic disk storage or another magnetic storage device. Certainly, a person skilled in the art may know that the computer storage medium is not limited to the foregoing types. The system memory 1004 and the mass storage device 1007 may be collectively referred to as a memory.

The memory stores one or more programs, and the one or more programs are configured to be executed by one or more CPUs 1001. The one or more programs include instructions used for implementing the foregoing method, and the CPU 1001 executes the one or more programs to implement the method provided in the foregoing method embodiments.

According to the various embodiments of this application, the computer device 1000 may further be connected, through a network such as the Internet, to a remote computer on the network for running. That is, the computer device 1000 may be connected to a network 1012 by a network interface unit 1011 connected to the system bus 1005, or may be connected to another type of network or a remote computer system (not shown) by a network interface unit 1011.

The memory further includes one or more programs. The one or more programs are stored in the memory and include operations to be executed by the computer device in the method provided in the embodiments of this application.

An embodiment of this application further provides a computer-readable storage medium, having at least one instruction, at least one program, a code set, or an instruction set stored therein, the at least one instruction, the at least one program, the code set, or the instruction set being loaded and executed by a processor to implement the medical image segmentation method according to any one of the foregoing embodiments.

An embodiment of this application provides a computer program product or a computer program. The computer program product or the computer program includes computer instructions, the computer instructions being stored in a computer-readable storage medium. A processor of a computer device reads the computer instructions from the computer-readable storage medium, and executes the computer instructions, to cause the computer device to perform the medical image segmentation method provided in the foregoing aspects.

A person of ordinary skill in the art may understand that all or some of the operations of the methods in the foregoing embodiments may be implemented by a program instructing relevant hardware. The program may be stored in a computer-readable storage medium. The computer-readable storage medium may be the computer-readable storage medium included in the memory in the foregoing embodiment, or may be a computer-readable storage medium that exists independently and that is not assembled in a terminal. The computer-readable storage medium stores at least one instruction, at least one program, a code set or an instruction set, the at least one instruction, the at least one program, the code set, or the instruction set being loaded or executed by the processor to implement the medical image segmentation method according to any one of the foregoing method embodiments.

In some embodiments, the computer-readable storage medium may include: a ROM, a RAM, a solid state drive (SSD), an optical disc, or the like. The RAM may include a resistance random access memory (ReRAM) and a dynamic random access memory (DRAM).

**Claims**

1. A medical image segmentation method, performed by a computer device, the method comprising:

   performing image segmentation on a sample medical image through a source domain segmentation model, to obtain a first segmentation result, the source domain segmentation model being obtained through training based on medical image data in a source domain, the sample medical image being an unannotated medical image in a target domain, a distribution of medical image data of the sample medical image in the target domain and a distribution of medical image data in the source domain being different;
   performing image segmentation on the sample medical image through a target domain segmentation model, to obtain a second segmentation result, wherein initial model parameters of the target domain segmentation model are the same as model parameters of the source domain segmentation model;
   adjusting the first segmentation result based on the second segmentation result and a segmentation confidence level of the target domain segmentation model, to obtain an adjusted segmentation result; and
   training the target domain segmentation model based on the second segmentation result and the adjusted segmentation result so as to update model parameters of the target domain segmentation model.

2. The method according to claim 1, wherein the adjusting the first segmentation result based on the second segmentation result and a segmentation confidence level of the target domain segmentation model, to obtain an adjusted segmentation result comprises:

   determining a label error indication map corresponding to the first segmentation result based on the second segmentation result, the label error indication map being configured for indicating correct pixel categories that are correctly labeled and incorrect pixel categories that are incorrectly labeled in the first segmentation result, pixel categories of pixels in the sample medical image being indicated in the first segmentation result; and
   adjusting, based on the segmentation confidence level, the incorrect pixel categories indicated in the label error indication map, to obtain the adjusted segmentation result.

3. The method according to claim 2, wherein the determining a label error indication map corresponding to the first segmentation result based on the second segmentation result comprises:

   determining, based on the first segmentation result, first pixels belonging to a first category;
   determining, based on the second segmentation result, potential probabilities that the first pixels belong to a second category, the second category being a pixel category different from the first category; and
   determining, based on the potential probabilities, the label error indication map corresponding to the first category in the first segmentation result.

4. The method according to claim 3, wherein the determining, based on the first segmentation result, first pixels belonging to a first category comprises:

   determining, based on the first segmentation result, probabilities that the pixels in the sample medical image belong to the first category;
   determining a first probability threshold corresponding to the first category based on the probabilities that the pixels belong to the first category; and
   determining, in a case that a probability that a pixel belongs to the first category is greater than the first probability threshold, the pixel as a first pixel.

5. The method according to claim 4, wherein the determining a first probability threshold corresponding to the first category based on the probabilities that the pixels belong to the first category comprises:

determining a maximum probability value in the probabilities that the pixels belong to the first category; and determining the first probability threshold based on the maximum probability value.

6. The method according to claim 3, wherein the determining, based on the potential probabilities, the label error indication map corresponding to the first category in the first segmentation result comprises:

determining a second probability threshold of the second category based on probabilities that the pixels belong to the second category in the second segmentation result;
determining a joint distribution matrix based on the potential probabilities and the second probability threshold, the joint distribution matrix being configured for indicating probabilities that pixels are labeled as the first category and a potential category of the pixels is the second category; and
determining the label error indication map corresponding to the first category based on the joint distribution matrix.

7. The method according to claim 6, wherein the determining a joint distribution matrix based on the potential probabilities and the second probability threshold comprises:

determining a confidence joint matrix based on the potential probabilities and the second probability threshold, the confidence joint matrix being configured for indicating a quantity of the first pixels of which the potential category is the second category; and
performing normalization processing on the confidence joint matrix, to obtain the joint distribution matrix.

8. The method according to claim 6, wherein the determining the label error indication map corresponding to the first category based on the joint distribution matrix comprises:

determining a pixel label error quantity based on the joint distribution matrix and a total quantity of the pixels in the sample medical image;
selecting pixels with incorrect labels from the pixels in the sample medical image based on the pixel label error quantity, probabilities that the pixels with incorrect labels belong to the first category being lower than probabilities that other pixels belong to the first category; and
performing first labeling on the pixels with incorrect labels and performing second labeling on the other pixels, to obtain the label error indication map.

9. The method according to any one of claims 2 to 8, wherein before the adjusting, based on the segmentation confidence level, the incorrect pixel categories indicated in the label error indication map, to obtain the adjusted segmentation result, the method further comprises:

performing n forward inferences on the sample medical image through the target domain segmentation model in a case that random perturbation is turned on, to obtain n pixel categories of the pixels in the sample medical image;
determining a model uncertainty map of the target domain segmentation model based on the n pixel categories of the pixels; and
determining, based on the model uncertainty map, the segmentation confidence level of the target domain segmentation model for segmenting pixels corresponding to the incorrect pixel categories.

10. The method according to claim 9, wherein the adjusting, based on the segmentation confidence level, the incorrect pixel categories indicated in the label error indication map, to obtain the adjusted segmentation result comprises:

determining an adjustment weight based on the segmentation confidence level for the pixels corresponding to the incorrect pixel categories, the adjustment weight being negatively correlated with the segmentation confidence level; and
adjusting the incorrect pixel categories based on the adjustment weight, to obtain the adjusted segmentation result.

11. The method according to any one of claims 1 to 8, wherein the training the target domain segmentation model based on the second segmentation result and the adjusted segmentation result so as to update model parameters of the target domain(404) comprises:

determining pixel category probabilities of the pixels based on the second segmentation result;

determining cross-entropy losses based on the pixel category probabilities and adjusted category probabilities of the pixels indicated in the adjusted segmentation result; and

training the target domain segmentation model based on the cross-entropy losses so as to update model parameters of the target domain segmentation model.

12. A medical image segmentation apparatus, comprising:

an image segmentation module, configured to perform image segmentation on a sample medical image through a source domain segmentation model, to obtain a first segmentation result, the source domain segmentation model being obtained through training based on medical image data in a source domain, the sample medical image being an unannotated medical image in a target domain, a data distribution of medical image data of the sample medical image in the target domain and a distribution of medical image data in the source domain being different;

the image segmentation module being further configured to perform image segmentation on the sample medical image through a target domain segmentation model, to obtain a second segmentation result, wherein initial model parameters of the target domain segmentation model are the same as model parameters of the source domain segmentation model;

an adjustment module, configured to adjust the first segmentation result based on the second segmentation result and a segmentation confidence level of the target domain segmentation model, to obtain an adjusted segmentation result; and

a training module, configured to train the target domain segmentation model based on the second segmentation result and the adjusted segmentation result so as to update model parameters of the target domain segmentation model.

13. The apparatus according to claim 12, wherein the adjustment module is further configured to:

determine a label error indication map corresponding to the first segmentation result based on the second segmentation result, the label error indication map being configured for indicating correct pixel categories that are correctly labeled and incorrect pixel categories that are incorrectly labeled in the first segmentation result, pixel categories of pixels in the sample medical image being indicated in the first segmentation result; and

adjust, based on the segmentation confidence level, the incorrect pixel categories indicated in the label error indication map, to obtain the adjusted segmentation result.

14. The apparatus according to claim 13, wherein the adjustment module is further configured to:

determine, based on the first segmentation result, first pixels belonging to a first category;

determine, based on the second segmentation result, potential probabilities that the first pixels belong to a second category, the second category being a pixel category different from the first category; and

determine, based on the potential probabilities, the label error indication map corresponding to the first category in the first segmentation result.

15. The apparatus according to claim 14, wherein the adjustment module is further configured to:

determine, based on the first segmentation result, probabilities that the pixels in the sample medical image belong to the first category;

determine a first probability threshold corresponding to the first category based on the probabilities that the pixels belong to the first category; and

determine, in a case that a probability that a pixel belongs to the first category is greater than the first probability threshold, the pixel as a first pixel.

16. The apparatus according to claim 15, wherein the adjustment module is further configured to:

determine a maximum probability value in the probabilities that the pixels belong to the first category; and

determine the first probability threshold based on the maximum probability value.

**17.** The apparatus according to claim 14, wherein the adjustment module is further configured to:

determine a second probability threshold of the second category based on probabilities that the pixels belong to the second category in the second segmentation result;

determine a joint distribution matrix based on the potential probabilities and the second probability threshold, the joint distribution matrix being configured for indicating probabilities that pixels are labeled as the first category and a potential category of the pixels is the second category; and

determine the label error indication map corresponding to the first category based on the joint distribution matrix.

**18.** A computer device, comprising a processor and a memory, the memory having at least one program stored therein, the at least one program being loaded and executed by the processor to implement the medical image segmentation method according to any one of claims 1 to 11.

**19.** A computer-readable storage medium, having at least one program stored therein, the at least one program being loaded and executed by a processor to implement the medical image segmentation method according to any one of claims 1 to 11.

**20.** A computer program product, comprising computer instructions, the computer instructions being stored in a computer-readable storage medium, a processor of a computer device reading the computer instructions from the computer-readable storage medium, and the processor executing the computer instructions to implement the medical image segmentation method according to any one of claims 1 to 11.

```
┌──────────────────┐                    ┌──────────────────┐
│  Source domain   │───────────────────▶│ First segmentation│
│segmentation model│                    │    result 103    │
│       102        │                    └──────────────────┘
└──────────────────┘                             │
       ▲                                          ▼
┌──────────┐        ┌──────────────────┐  ┌──────────────────┐
│ Sample   │        │   Segmentation   │─▶│    Adjusted      │
│ medical  │───────▶│confidence level 106│ │segmentation result│──┐
│ image    │        └──────────────────┘  │      107         │  ¦
│  101     │               ▲              └──────────────────┘  ¦
└──────────┘               │                     ▲              ¦
       │           ┌──────────────────┐  ┌──────────────────┐  ¦
       └──────────▶│  Target domain   │─▶│Second segmentation│──┤
                   │segmentation model│  │    result 105    │  ¦
                   │       104        │  └──────────────────┘  ¦
                   └──────────────────┘                        ¦
                          ▲           Training to              ¦
                          ¦             update                 ¦
                          └─────────────────────────────────────┘
```

## FIG. 1

Source domain
image data

Source domain
segmentation model

Only the source domain
segmentation model is
provided, and no source
domain image data is provided

Source domain
segmentation model

Target domain
segmentation model

Unannotated target
domain image data

## FIG. 2

_310

| Target image |
| --- |

Target image →

_320

Target domain segmentation model

FIG. 3

| Perform image segmentation on a sample medical image through a source domain segmentation model, to obtain a first segmentation result, the source domain segmentation model being obtained through training based on medical image data in a source domain, the sample medical image being an unannotated medical image in a target domain, data distributions of medical images in the target domain and the source domain being different | _401 |
| --- | --- |

↓

| Perform image segmentation on the sample medical image through a target domain segmentation model, to obtain a second segmentation result | _402 |
| --- | --- |

↓

| Adjust the first segmentation result based on the second segmentation result and a segmentation confidence level of the target domain segmentation model, to obtain an adjusted segmentation result | _403 |
| --- | --- |

↓

| train the target domain segmentation model based on the second segmentation result and the adjusted segmentation result so as to update model parameters of the target domain segmentation model | _404 |
| --- | --- |

FIG. 4

Perform image segmentation on a sample medical image through a source domain segmentation model, to obtain a first segmentation result — 501

Perform image segmentation on the sample medical image through a target domain segmentation model, to obtain a second segmentation result — 502

Determine a label error indication map corresponding to the first segmentation result based on the second segmentation result, the label error indication map being configured for indicating correct pixel categories that are correctly labeled and incorrect pixel categories that are incorrectly labeled in the first segmentation result, pixel categories of pixels in the sample medical image being indicated in the first segmentation result — 503

Adjust, based on the segmentation confidence level, the incorrect pixel categories indicated in the label error indication map, to obtain the adjusted segmentation result — 504

train the target domain segmentation model based on the second segmentation result and the adjusted segmentation result so as to update model parameters of the target domain segmentation model — 505

FIG. 5

Perform image segmentation on a sample medical image through a source domain segmentation model, to obtain a first segmentation result — 601

Perform image segmentation on the sample medical image through a target domain segmentation model, to obtain a second segmentation result — 602

Determine a label error indication map corresponding to the first segmentation result based on the second segmentation result — 603

Perform n forward inferences on the sample medical image through the target domain segmentation model in a case that random perturbation is turned on, to obtain n pixel categories of the pixels in the sample medical image — 604

Determine a model uncertainty map of the target domain segmentation model based on the n pixel categories of the pixels — 605

Determine, based on the model uncertainty map, the segmentation confidence level of the target domain segmentation model for segmenting pixels corresponding to the incorrect pixel categories — 606

Determine an adjustment weight based on the segmentation confidence level for the pixels corresponding to the incorrect pixel categories, the adjustment weight being negatively correlated with the segmentation confidence level — 607

Adjust the incorrect pixel categories based on the adjustment weight, to obtain the adjusted segmentation result — 608

Determine pixel category probabilities of the pixels based on the second segmentation result — 609

Determine cross-entropy losses based on the pixel category probabilities and adjusted category probabilities of the pixels indicated in the adjusted segmentation result — 610

Train the target domain segmentation model based on the cross-entropy losses so as to update model parameters of the target domain segmentation model — 611

FIG. 6

FIG. 7

FIG. 8

FIG. 9

FIG. 10

## INTERNATIONAL SEARCH REPORT

| International application No. |
| --- |
| **PCT/CN2023/084721** |

**A.    CLASSIFICATION OF SUBJECT MATTER**

G06V 10/764(2022.01)i;  G06T 7/10(2017.01)i

According to International Patent Classification (IPC) or to both national classification and IPC

**B.    FIELDS SEARCHED**

Minimum documentation searched (classification system followed by classification symbols)

IPC: G06V,  G06T,  G06N

Documentation searched other than minimum documentation to the extent that such documents are included in the fields searched

Electronic data base consulted during the international search (name of data base and, where practicable, search terms used)

CJFD, VEN, DWPI, CNABS, CNTXT, WOTXT, EPTXT, USTXT, CNKI: 置信度, 源域, 样本域, 模型迁移, 图像分割, 校准, 校正, 无源, 无监督, 域适应, 交叉熵, 数据分布, 医疗图像, unsupervised, segmentation, source domain, target domain

**C.    DOCUMENTS CONSIDERED TO BE RELEVANT**

| Category* | Citation of document, with indication, where appropriate, of the relevant passages | Relevant to claim No. |
| --- | --- | --- |
| PX | CN 115115829 A (TENCENT MEDICAL HEALTH SHENZHEN CO., LTD.) 27 September 2022 (2022-09-27)<br>claims 1-15, description, paragraphs 22-234, and figures 1-10 | 1-20 |
| X | CN 112419326 A (TENCENT TECHNOLOGY (SHENZHEN) CO., LTD.) 26 February 2021 (2021-02-26)<br>claims 1-8, and description, paragraphs 70-193 | 1-20 |
| A | CN 108062753 A (CHONGQING UNIVERSITY OF TECHNOLOGY) 22 May 2018 (2018-05-22)<br>entire document | 1-20 |
| A | CN 111476771 A (SUN YAT-SEN UNIVERSITY) 31 July 2020 (2020-07-31)<br>entire document | 1-20 |
| A | CN 113822851 A (TENCENT TECHNOLOGY (SHENZHEN) CO., LTD.) 21 December 2021 (2021-12-21)<br>entire document | 1-20 |

☑ Further documents are listed in the continuation of Box C.     ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **07 June 2023** | **20 June 2023** |

| Name and mailing address of the ISA/CN | Authorized officer |
| --- | --- |
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/084721**

**C.    DOCUMENTS CONSIDERED TO BE RELEVANT**

| Category* | Citation of document, with indication, where appropriate, of the relevant passages | Relevant to claim No. |
|---|---|---|
| A | AU 2020103905 A4 (CHONGQING NORMAL UNIVERSITY) 11 February 2021 (2021-02-11)<br>entire document | 1-20 |

Form PCT/ISA/210 (second sheet) (July 2022)

**INTERNATIONAL SEARCH REPORT**
Information on patent family members

International application No.

**PCT/CN2023/084721**

| Patent document cited in search report | | | Publication date (day/month/year) | Patent family member(s) | | | Publication date (day/month/year) |
|---|---|---|---|---|---|---|---|
| CN | 115115829 | A | 27 September 2022 | None | | | |
| CN | 112419326 | A | 26 February 2021 | CN | 112419326 | B | 23 May 2023 |
| CN | 108062753 | A | 22 May 2018 | CN | 108062753 | B | 17 April 2020 |
| CN | 111476771 | A | 31 July 2020 | None | | | |
| CN | 113822851 | A | 21 December 2021 | None | | | |
| AU | 2020103905 | A4 | 11 February 2021 | None | | | |

Form PCT/ISA/210 (patent family annex) (July 2022)

**EP 4 447 000 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210486701